(19) Europäisches Patentamt
European Patent Office
Office européen des brevets

(11) Numéro de publication: **0 012 052**
**B1**

(12) **FASCICULE DE BREVET EUROPEEN**

(45) Date de publication du fascicule du brevet: **02.06.82**

(21) Numéro de dépôt: **79400839.1**

(22) Date de dépôt: **09.11.79**

(51) Int. Cl.³: **B 01 D 19/04,**
C 07 C 121/32,
C 08 G 65/28, C 11 D 1/722

(54) **Procédé de régulation des mousses dans les installations de fabrication de l'acrylonitrile et des esters acryliques.**

(30) Priorité: **30.11.78 FR 7833781**

(43) Date de publication de la demande:
**11.06.80 Bulletin 80/12**

(45) Mention de la délivrance du brevet:
**02.06.82 Bulletin 82/22**

(84) Etats contractants désignés:
**DE FR GB IT NL**

(56) Documents cités:
**DE - A - 2 252 186**
**DE - A - 2 724 349**
**FR - A - 2 247 532**
**GB - A - 800 159**
**GB - A - 1 097 491**
**US - A - 4 105 582**

(73) Titulaire: **P C U K PRODUITS CHIMIQUES UGINE KUHLMANN**
**Service Propriété Industrielle Tour Manhattan**
**F-92087 Paris La Defense 2 Cédex 21 (FR)**

(72) Inventeur: **Bruno, Francis**
**11, rue Marbeau**
**F-75116 Paris (FR)**
Inventeur: **Konareff, Jean**
**Route du Bas Privas Charly**
**F-69390 Vernaison (FR)**
Inventeur: **Pardon, Louis**
**28, rue du 11 Novembre 1918**
**F-69230 Saint-Genis Laval (FR)**

(74) Mandataire: **Houssin, Jean et al,**
**PRODUITS CHIMIQUES UGINE KUHLMANN**
**Service Propriété Industrielle Tour Manhattan**
**Cedex 21**
**F-92087 Paris La Defense 2 (FR)**

**EP 0 012 052 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procédé de régulation des mousses dans les installations de fabrication de l'acrylonitrile et des esters acryliques

La présente invention concernce un procédé de limitation et de régulation des mousses dans les installations industrielles de fabrication de l'acrylonitrile et des dérivés acryliques.

L'acrylonitrile est un produit industriel utilisé entre autres applications pour la fabrication de matières plastiques de polymères de poids moléculaire élevés servant d'agents dispersants ou épaississants, de fibres artificielles... En raison de son intérêt, des procédés modernes de fabrication, mettant en oeuvre des matières premières bon marché tels l'ammoniac et le propylène, ont fait leur apparition durant ces dernières années. Bien qu'il existe des différences importantes entre les procédés actuellement utilisés, ils sont tous du même type. Du propylène et de l'ammoniac sont vaporisés puis mélangés avec de l'air et introduits dans un réacteur contenant une masse catalytique appropriée, lit fixe ou lit fluidisé suivant les procédés. Les gaz sortant du réacteur sont refroidis puis envoyés dans un premier absorbeur où l'ammoniac non converti est transformé par l'acide sulfurique en sulfate d'ammonium. Les gaz sont ensuite lavés à l'eau, de façon à obtenir une solution diluée d'acrylonitrile; cette solution, après concentration dans une colonne d'épuisement, conduit à l'acrylonitrile brut.

Cet acrylonitrile brut est purifié, dans une série de colonnes à distiller qui permettent de récupérer des sous-produits intéressants sur le plan industriel, tels l'acide cyanhydrique et l'acétonitrile.

Tous les procédés actuels exigent après l'étape de réaction que soient effectuées différentes opérations de séparation par absorption et distillation. Le milieu réactionnel contenant des produits très divers et étant soumis à de fortes agitations il se produit des phénomènes de moussage dans les installations qui, s'ils ne sont pas correctement contrôlés, ont des effets néfastes sur l'efficacité de la séparation et par conséquent sur la rentabilité globale du procédé.

De même dans le procédé de fabrication des esters acryliques à partir de l'acrylonitrile on effectue des réactions d'hydrolyse et d'estérification en milieu sulfurique à température élevée. Dans les deux cas le milieu réactionnel est soumis à de fortes agitations et il se produit des phénomènes de moussage qu'il est nécessaire de contrôler.

Pour limiter et contrôler ces phénomènes de moussage il est connu d'utiliser comme agents régulateurs des mousses des produits siliconés. Mais ces produits présentent l'inconvénient d'être difficiles à disperser dans l'eau, ce qui rend leur emploi malaisé, et d'être partiellement entrainés par la vapeur d'eau, ce qui rend leur utilisation coûteuse. D'autres compositions, en particulier celles du brevet américain 3.657.136 ont également été proposées dans le même but, mais elles présentent l'inconvénient d'être constituées d'un mélange de produits de nature très différente ce qui entraine des opérations de mélange à chaud délicates et partant coûteuses, et d'autre part de nécessiter la présence d'un agent tensio-actif pour faciliter la distribution de façon uniforme et rapide du mélange dans l'installation ce qui exige une seconde opération de mélange avant introduction dans l'installation.

La demanderesse a constaté que l'emploi de mélanges de tensio-actifs de formule générale:

$$R—(OCH_2CH_2)_n\,(OCH_2CHCH_3)_m\,OR' \qquad (I)$$

dans laquelle R est un groupement alkyle contenant 8 à 18 atomes de carbone, R' est un groupe alkyle ou benzyle, n un nombre compris entre 2 et 20, m un nombre compris entre 4 et 20, est particulièrement intéressant pour limiter et contrôler la formation de mousses dans les procédés de préparation d'acrylonitrile et d'esters acryliques.

Dans l'état actuel de la technique, il n'est pas possible d'isoler les divers produits de formule (I), et l'on obtient dans leur fabrication à partir d'alcool ou de mélanges d'alcools un mélange de composés (I) dont les formules se distribuent à l'intérieur de la formule générale (I). On désignera ces produits par l'expression "Produits I" étant entendu qu'il s'agit de mélanges de produits définis ~~par leur procédé de prépa~~ration.

Les doses des produits I à utiliser sont fonction des conditions d'exploitation, et sont généralement de préférence compris entre 50 et 500 ppm par rapport aux produits acryliques obtenus.

Les produits I peuvent être préparés par exemple par éthoxylation—propoxylation d'alcools suivie d'une alkylation. Dans ce cas on introduit dans un réacteur contenant un alcool ROH, ou un mélange d'alcools, un mélange d'oxyde d'éthylène et d'oxyde de propylène ou successivement l'un et l'autre de ces oxydes, puis on ajoute les quantités nécessaires de sodium ou de soude et/ou d'alcoolate de sodium pour la préparation de l'alcoolate d'alcool oxyéthyléoxypropylé. On introduit enfin un halogénure de méthyle R'X, chlorure ou bromure, et on laisse réagir.

Les exemples ci-dessus illustrent de façon non limitative la préparation des compositions utilisées dans l'invention et le procédé de régulation des mousses des installations de fabrication de l'acrylonitrile et des esters acryliques selon l'invention.

## Exemple 1

Préparation d'une composition selon la formule générale I.

On prépare un mélange de produits de formule:

$$R—(OCH_2CH_2)_n\ (OCH_2CHCH_3)_m\ OCH_3$$

dans laquelle R est un groupement alkyle contenant 9 à 11 atomes de carbone, n un nombre voisin de 5 et m un nombre voisin de 8, de la façon suivante: 0,8 g d'hydroxyde de sodium en paillettes sont ajoutés à 138 g d'un mélange 50/50 d'alcools oxo $C_9—C_{11}$. Après étêtage sous vide on introduit à 140—150°C 612 g d'un mélange d'oxyde d'éthylène et d'oxyde de propylène contenant 33% d'oxyde d'éthylène. On ajoute ensuite la quantité nécessaire d'hydroxyde de sodium, avec un exçès de 10%, pour la préparation de l'alcoolate d'alcool oxyéthylé-oxypropylé. On introduit en 12 heures 60,5 g chlorure de méthyle et on laisse réagir 12 heures à 150°C. Après refroidissement on additionne 600 g d'eau. Le produit obtenu se présente après lavage sous l'aspect d'un liquide limpide ou légèrement trouble de couleur jaune paille.

Sa viscosité à 25°C est de 68 centipoises environ, son point de coulage de −58°C, sa masse volumique égale à 979 kg/m³. Le produit est insoluble dans l'eau quelle que soit la température, mais il se disperse facilement dans ce milieu sous faible agitation.

Son pouvoir moussant déterminé selon la méthode de la norme française NF T 73.404 est nul, quelle que soit la concentration, pour des températures allant de 15 à 75°C.

Cette composition présente le grande intérêt de n'être absolument pas entrainable à la vapeur d'eau à l'inverse des antimousses siliconés dont l'emploi est souvent proposé pour le même usage comme le montrent les essais suivants:

Une solution de sulfate d'ammonium à 50% contenant 0,5 g/l de la composition selon l'exemple 1 est distillée dans une installation de laboratoire sous une pression de 200 mm de mercure à la température de 100°C (température dans le bouilleur). Le distillat recueilli est limpide et contient moins de 100 ppm de carbone. Dans les mêmes conditions, un produit antimousse siliconé (composé organosilicique) conduit à la récupération d'un distillat trouble, et en fin d'opération 10% de l'antimousse siliconé est passé dans le distillat.

Exemple 2

Préparation d'une composition selon la formule générale I.

On prépare un mélange de produits de formule:

$$R(OCH_2CH_2)_n\ (OCH_2CHCH_3)_m\ OCH_3$$

dans laquelle R est un groupement alkyle contenant de 13 à 15 atomes de carbone, n un nombre voisin de 9 et m un nombre voisin de 7 de la façon suivante: 0,8 g d'hydroxyde de sodium en paillettes sont ajoutés à 150 g d'un mélange d'alcools oxo $C_{13}—C_{15}$. Après étêtage sous vide on introduit à 123—125°C, 594 g d'un mélange d'oxyde d'éthylène et d'oxyde de propylène contenant 50% d'oxyde d'éthylène. On ajoute ensuite la quantité nécessaire d'hydroxyde de sodium avec un excès de 10% pour la préparation de l'alcoolate d'alcool oxyéthylé-oxypropylé. On laisse réagir 6 heures à 145°C avant d'étêter sous pression réduite. La température est ramenée à 125°C et on introduit en 4 heures 30 minutes 68 g de chlorure de méthyle. On laisse réagir 2 heures à 125°C.

Aprés refroidissement, on additionne 180 g d'eau. Le produit est décanté et lavé une seconde fois avec 75 g d'eau puis séché sous pression réduite à 145°C.

Il se présente sous l'aspect d'un liquide limpide ou légèrement trouble de couleur jaune paille. Sa température de trouble à la concentration de 1% dans l'eau déterminée suivant la méthode de la norme T 73.403 est de 29°C environ.

Exemple 3

Dans un atelier de fabrication de l'acrylonitrile par oxydation du propylène par l'air, les gaz de réaction sont envoyés, après refroidissement vers 160—230°C dans une tour de lavage acide qui retient l'ammoniac non transformé, refroidit les gaz vers 90°C, produit une solution de sulfate d'ammonium à 40% et sépare une certaine quantité de polymères.

Les conditions de fonctionnement de cette colonne, température 90—95°C, concentration de la solution de sulfate d'ammonium 40%, concentration d'acide sulfurique 1,8 à 2%, ainsi que la nature des produits présents sont particulièrement favorables à la formation de mousses, et en l'absence d'agent régulateur, le travail de la colonne est incontrôlable.

On injecte en tête de la colonne qui traite 6 t/H d'acrylonitrile, 120 kg/h d'une solution aqueuse à 1,3% en poids de la composition préparée à l'exemple 1. La colonne fonctionne de façon satisfaisante en l'absence de toute mousse.

Exemple 4

Dans un atelier de fabrication d'acrylonitrile, l'acrylonitrile technique obtenu après séparation de la cyanhydrine d'acroléine et de l'acide cyanhydrique par distillation est envoyé dans une colonne de distillation destinée à séparer l'acétonitrile. La séparation acétonitrile-acrylonitrile est réalisée par distillation extractive en présence d'eau, cette eau étant introduite en tête de colonne. On élimine le propionitrile et les nitriles supérieurs par soutirage au niveau des plateaux du bas de la colonne.

Les conditions de fonctionnement de cette colonne, température 67—68°C en tête de colonne, 100°C en pied de colonne, concentration en acétonitrile de 200 ppm en tête de

colonne à 0,5% en pied, ainsi que la nature des produits mis en oeuvre sont particulièrement favorables à la formation de mousses, et en l'absence d'agent régulateur le travail de la colonne est incontrôlable.

On injecte en tête de la colonne qui traite 6 t/h d'acrylonitrile, 30 kg/h d'une solution aqueuse à 1,3% en poids de la composition préparée à l'exemple 1. La colonne fonctionne parfaitement en l'absence de toute mousse.

Exemple 5

Dans un atelier de fabrication d'acrylonitrile, les gaz de réaction sont envoyés, après l'élimination de l'ammoniac dans une colonne de lavage et de refroidissement, dans une colonne d'absorption. Le lavage à l'eau qui s'effectue dans cette colonne permet de retenir l'acrylonitrile et les autres produits absorbables à l'état de solution aqueuse à 4% en poids d'acrylonitrile.

Les conditions de marche de la colonne, température 6°C en tête et 20°C en pied et les fluides utilisés créent des mousses gênantes pour le bon fonctionnement, et en l'absence d'agent régulateur, le travail de la colonne est incontrôlable.

On injecte en tête de la colonne qui traite 6 t/h d'acrylonitrile, 32 kg/h d'une solution aqueuse à 1,3% en poids de la composition préparée à l'exemple 1. La colonne fonctionne de façon satisfaisante en l'absence de toute mousse.

Exemple 6

Dans un atelier de fabrication de méthacrylate de méthyle par réaction à chaud entre l'acrylonitrile, l'acide sulfurique et l'éthanol (hydrolyse puis estérification) il peut se produire des réactions de polymérisation qui sont activées par les phénomènes de moussage. En l'absence d'agent de régulation de la mousse les stabilisants introduits pour lutter contre les polymérisations parasites se révèlent peu efficaces. L'introduction de 200 kg/h d'une solution aqueuse à 2% en poids de la composition préparée à l'exemple 1 dans le réacteur traitant 6 t/h de méthacrylate de méthyle permet d'en assurer le bon fonctionnement en limitant les réactions de polymérisation à un taux acceptable et en limitant et régulant la mousse formée.

**Revendications**

1. Procédé de régulation des mousses dans les installations de fabrication de l'acrylonitrile et des esters acryliques caractérisé par l'utilisation au stade de la purification de ces produits de mélanges de tensio-actifs non ioniques de formule:

$$R\text{—}(OCH_2CH_2)_n\,(OCH_2CHCH_3)_m\,OR' \quad (I)$$

dans laquelle R est un groupement alkyle contenant 8 à 18 atomes de carbone, R' est un groupe alkyle ou benzyle, n un nombre compris entre 2 et 20, m un nombre compris entre 4 et 20.

2. Procédé selon la revendication 1 où les tensio-actifs utilisés ont pour formule:

$$R\text{—}(OCH_2CH_2)_n\,(OCH_2CHCH_3)_m\,OCH_3 \quad (II)$$

dans laquelle R est un groupement alkyle contenant 9 à 11 atomes de carbone, n un nombre voisin de 5 et m un nombre voisin de 8.

**Claims**

1. Process for controlling the foams in installations for the manufacture of acrylonitrile and acrylic acid esters, characterised by the use, at the stage of purification of these products, of mixtures of non-ionic surface-active agents of the formula:

$$R\text{—}(OCH_2CH_2)_n(OCH_2CHCH_3)_mOR' \quad (I)$$

in which R is an alkyl group containing 8 to 18 carbon atoms, R' is an alkyl or benzyl group, n is a number between 2 and 20 and m is a number between 4 and 20.

2. Process according to Claim 1, in which the surface-active agents used have the formula:

$$R\text{—}(OCH_2CH_2)_n(OCH_2CHCH_3)_mOCH_3 \quad (II)$$

in which R is an alkyl group containing 9 to 11 carbon atoms, n is a number of the order of 5 and m is a number of the order of 8.

**Patentansprüche**

1. Verfahren zur Schaumregelung in Anlagen zur Herstellung von Acrylnitril und Acrylsäureestern, dadurch gekennzeichnet, daß man bei der Reinigung dieser Produkte Gemische von nicht-ionischen Tensiden der Formel

$$R\text{—}(OCH_2CH_2)_n\text{—}(OCH_2CHCH_3)_mOR' \quad (I)$$

verwendet, in der R eine Alkylgruppe mit 8 bis 18 Kohlenstoffatomen, R' eine Alkyl- oder Benzylgruppe, n eine Zahl von 2 bis 20 und m eine Zahl von 4 bis 20 bedeutet.

2. Verfahren nach Anspruch 1, wo die Tenside die Formel

$$R\text{—}(OCH_2CH_2)_n(OCH_2CHCH_3)_mOCH_3 \quad (II)$$

haben, in der R eine Alkylgruppe mit 9 bis 11 Kohlenstoffatomen, n eine ganze Zahl um 5 und m eine Zahl um 8 bedeutet.